Europäisches Patentamt

European Patent Office

Office européen des brevets

(19)

(11) Publication number: **0 210 178 B1**

(12) **EUROPEAN PATENT SPECIFICATION**
published in accordance with Art.
158(3) EPC

(45) Date of publication of patent specification: 07.08.91 (51) Int. Cl.⁵: **A61F 5/00**

(21) Application number: 85905368.8

(22) Date of filing: **17.10.85**

(86) International application number:
**PCT/US85/02047**

(87) International publication number:
**WO 86/04228 (31.07.86 86/17)**

(54) LEG BRACE FOR CONTROLLING SUBLUXATION.

(30) Priority: **22.01.85 US 693143**

(43) Date of publication of application:
**04.02.87 Bulletin 87/06**

(45) Publication of the grant of the patent:
**07.08.91 Bulletin 91/32**

(84) Designated Contracting States:
**CH DE FR GB LI NL SE**

(56) References cited:
US-A- 2 952 459    US-A- 3 826 251
US-A- 4 320 747    US-A- 4 361 142
US-A- 4 370 977    US-A- 4 379 463
US-A- 4 407 276    US-A- 4 428 369
US-A- 4 487 200    US-A- 4 489 718
US-A- 4 494 534    US-A- 4 554 913

(73) Proprietor: **MEDICAL DESIGNS, INC.**
**P.O. Box 918**
**Azle, TX 76020(US)**

(72) Inventor: **FORD, Edward, I.**
**P.O. Box 918**
**Azle, TX 76020(US)**

(74) Representative: **Schmitz, Jean-Marie et al**
**OFFICE DENNEMEYER S.à.r.l. P.O. Box 1502**
**L-1015 Luxembourg(LU)**

## Description

This invention relates to a knee brace. More particularly, it relates to a hinged leg brace for alleviating problems with rotation and subluxation of a tibia with respect to a femur in the leg of a wearer.

A wide variety of approaches have been employed in the prior art for braces for legs and the like. Thus, for example, reference may be had to United States Patents Nos. 618,097; 1,228,113; 2,144,641; 2,308,776; 3,528,412; 3,581,741 3,669,105 for various types of knee braces for protection of knee joints and the like. None of these prior art braces have been totally satisfactory in preventing or alleviating problems with rotation of the tibia with respect to the femur as a primary problem; or subluxation of the tibia with respect to the femur. A germain invention was described and claimed in US-A-4,407,276 entitled "BRACE FOR ARTICULATED LIMBS", Bledsoe, issued Oct. 4, 1983, assigned to the assignee of this invention and the contents of that patent are incorporated herein by reference for details that are omitted herefrom.

In addition, applications on improvements have been filed by other co-workers and are US-A-4 489 718 entitled "KNEE BRACE HINGE", filed March 8, 1983 by Kelsey Martin and assigned to the assignee of this invention; US-A-4 487 208 "BRACE FOR KNEE", filed April 25, 1983 and Serial No. 511,265, "DEROTATION LEG BRACE", filed July 6, 1983. All delineated improvements directed to the hinge are to structure employing metal cuffs anteriorly of the tibia and posterior of the femur. The files of these patent applications show a good review of prior art in the United States patents and in general. A portion of that material will be repeated herein to give the reader an understanding without requiring reference to another instrument.

Doctors and other technicians frequently impose restraints on a person's bones, joints and connective tissue to allow natural healing to be started or completed before restraints are released or broadened. Broadening allows additional movement and alleviates problems with atrophying of the muscles or the like. The new thinking is apparently to eliminate as much as possible of the bulky conventional cast and to wear temporary apparatus that can be removed to prevent problems with skin maceration, to accomodate changes as healing of the injury takes place and to allow better attention to personal hygiene and the like.

In those file histories, also, were discussed groupings of knee braces and the like ranging from the immobilizers which did not have a hinge and are not discussed herein; through braces that are relatively permanent for long term wear such as those disclosed in US-A-2,632,440; 2,943,622; 3,826,251; 3,827,431; and 3,844,279. These long term braces did not have the rotation and subluxation control and cuffs of the improved inventions. Typical of the apparatus that employs the hinge with knee braces are those shown in the following United States patents: US-A-3,575,166 describes apparatus in which two rigid bodies, or covers, partially encircle a person's thigh and calf, respectively, encompassing about 270° of the wearer's leg member and employing a flexible elastomeric material to fill in the remaining 90° gap. A single hinge is rigidly connected on each side of the thigh and calf cuffs in order to provide some control with regard to a person's knee movements. US-A-3,581,741 discloses similar "body" portions 18, 28 which are described as being a tough polymeric plastic material which may be internally reinforced with glass fibers and the like. US-A-3,669,105 discloses a construction which has the advantage of being manufactured and worn by athletes having weakened knees. US-A-3,785,372 describes a relatively complicated hinge apparatus. US-A-3,786,804 describes apparatus having a single piece cylindrical sleeve of elastic material with loosely fitting pockets. US-A-4,220,148 discloses a stabilizer but does not disclose the aspects of this invention. US-A-4,233,967 discloses a plastic construction for attaching pairs of elongated braces but does not have the structure of this invention. US-A-4,241,730 discloses a knee support which includes a pair of pivotally interconnected rigid braces but does not include the structure of this invention; US-A-4,271,831 describes a knee brace having upper and lower sections that encase the proximal and distal members of the leg and have a hinge therebetween but do not have the structural features of this invention.

In the delineated prosecution histories there can be found references such as the following: US-A-3,826,251 describes a hinge with a locking joint that locks under weight but is pivotal when the weight is released; US-A-4,252,111 discloses a locking mechanism for locking a hinge through a movable pin that is inserted into mating apertures; US-A-4,353,361 discloses an orthotic/prosthetic joint for supporting an infirm anatomical knee while permitting normal motion. The structure contains end portions that are secured to parallel connecting members for independent movement with concealed stops disposed intermediate to end portions and having a reinforced corrugation 20 on the upper assembly with a large calf cuff of custom molded plastic in the lower assembly. US-A-4,361,142 discloses a knee orthosis and joint construction for protective treatment of ligaments during healing. There is a fairly complicated hinge structure with a single condyle pad and encircling straps.

Also, prosecution histories reveal the following United States patents: US-A-4,271,831 shows a knee brace with encircling straps for tying rigid bars to the front of the tibia and include a drift control device. US-A-3,581,741 describes a knee brace having an encircling upper rigid body portion and a lower rigid body portion with straps for controlling movement about the medial and lateral meniscus. US-A-4,387,709 describes a knee brace having a substantially complete, open-ended tubular shell in an integrally formed piece of semi-rigid material shaped to fit a particular leg adapted to be slipped on and off, and having straps to hold it closed. US-A-4,144,592 describes a knee guard for disposition along with the thigh of the user for protecting the knee with internal pads and having straps that hold a rigid front onto the wearer or the user as in a football uniform. US-A-4 370 977 describes a knee brace with identical side members and hinged members that control 30 to 60 degree flexion. The side members may be secured to the leg by inserting thigh straps and calf straps through suitable buckles. US-A-4 489 718 describes an improved knee brace hinge for controlling the degree of motion permitted by a wearer's knee.

In US-A-4 320 747 there is described a knee brace according to the preamble of claim 1. More specifically, US-A-4 320 747 describes a knee brace comprising first and second pairs of elongate braces with each of the braces being rotationally stiff so as to resist both torsion and bending forces, the first pair of braces being adapted to lie on opposite sides of the wearer's thigh and the second pair of braces being adapted to lie on opposite sides of the wearer's calf, a pair of respective hinge means connected respectively with the first and second pairs of braces at their respective central ends for allowing and controlling flexion and extension of the wearer's leg, and means for adjustably placing and holding respective pairs of elongate braces at desired attained positions on respective opposite sides of the wearer's thigh and calf with the hinge means positioned correctly adjacent the wearer's knee, the adjustable means comprising a plurality of flexible non-metallic straps connected to a plurality of respective attaching means anteriorly and posteriorly of the braces, a plurality of pads being disposed interiorly of and respectively connected with the straps (25) and the pairs of braces (13,15) for comfort of the wearer.

It has been found desirable to provide a knee brace that has the following features not heretofore provided by the prior art:

1. The knee brace should have flexible non-metallic straps that lay closer to the leg than the prior art.

2. The straps should have individual points of fixation, both front and back, of respective braces and hinge means to be individually adjusted so that one size can fit any leg; yet that can be tightened comfortably to control both subluxation and rotation.

3. The straps should be padded both anteriorly and posteriorly of the leg for comfort.

4. The brace should have dual condyle pads that are adapted to fit closely to support the condyle (bone knuckle) on both the femur and the tibia.

5. These condyle pads should be fully adjustable to any length or circumferential placement. In this way the condyle pads allow free movement without bunching of the condyle pads or introducing discomfort; yet, continue to support the condyle on their respective elements.

6. The knee brace hinge needs, in addition to non-stretch, flexible straps to compensate for laxity in ligaments, a hinge that has diverging slots to facilitate access to adjusting the control elements that control the degrees of freedom in flexion and extension.

7. It is desirable that the knee brace hinge have suitable covers such that it can be employed in sports or the like without endangering any other participants in the sport, as well as being safe for the wearer.

In the foregoing paragraph it can be seen that the prior art has not provided these desirable features.

Accordingly, it is an object of this invention to provide a knee brace that alleviates the deficiencies of the prior art, particularly with respect to the rotation and subluxation of the tibia with respect to the femur and that provides one or more of the features delineated hereinbefore as desirable and not heretofore provided by the prior art.

To achieve this, the knee brace of the invention is characterized by the features set forth in the characterizing portion of claim 1.

According to the invention, there is provided a plurality of eight inelastic straps that are fully adjustable and adapted to fit any size leg, a plurality of 16 attaching D-ring means for affixing respective straps, 8 said D-ring means being connected at the anterior of said braces above and below said hinges and 8 said D-ring means being connected at the posterior above and below said hinges, said D-ring means being connected with said braces and defining respective individual points for affixing said plurality of the 8 straps above and below said hinge means such that one size fits any leg, controls subluxation and distal migration and compensates for laxity of ligaments in the leg, and a plurality of two contoured condyle pads on each side interiorly of each respective said hinge means, each pair of said two condyle pads allowing each segment of the leg to flex independently of the other without bunching either said condyle pad with respect to said condyle of either the tibia of the femur; said condyle pads being shaped, respectively, to fit the supracondylar regions of the tibia and femur, respectively, such that they support the condyle regardless of flexion or extension of the tibia with respect to the femur.

Advantageous embodiments of the invention are claimed in the subclaims. It is a specific feature of this invention to provide a lightweight knee brace that can be readily removed or adjusted, and alleviates problems with swelling, muscle atrophy, and hygiene; as well as providing all of the features not heretofore provided by the prior art. These and other features will become apparent from the descriptive matter hereinafter, particularly when taken in conjunction with the appended drawings.

In preferred embodiments there are employed plastic intermediate the D-ring means and the braces with riveted construction; with shaped foam pads; with the covered hinge option for use in playing sports; and dual condyle pads on each side, each condyle pad being adapted to closely fit and support the condyle (bone knuckle) of the respective femur and tibia.

It is believed helpful to have a broad discussion of this invention before considering the detailed aspects. The knee brace attaches to the thigh proximally with both anterior and posterior inelastic straps that coact with the plastic connected to the braces to encompass the posterior, medial and lateral aspects of the thigh. A soft liner is employed to facilitate pressure against the skin. Specifically, the straps are preferably elongate straps with Velcro attachments to be able to be affixed at any length. In this fashion the thigh is grasped firmly. Descending from the proximal thigh are the braces which run to the hinge and thence to the calf. The hinge facilitates adjustment through a particular construction and allows controlled flexion and extension of the knee joint as discussed in more detail hereinafter. The braces may be contoured to the shape of a leg either right or left such that one size fits all in a truly universal design. The braces are hinged with a hinge that has a cam design which allows the brace to flex and extend simulating the normal articulated cam effect of a normal knee joint. On the inner aspect of each hinge are a pair of respective condyle pads on each side. The condyle pads are made of soft elastic material, such as foam material to rest against the medial and lateral femoral condyle of the knee and hold the brace in place and the condyles at their respective desired positions. The straps run above the patella across the front of the knee and the lower straps run below the level of the patella at the level of the tibial tubercle of the insertion of the patella tendon. This lower strap has been moved inferiorly so that flexion and extension does not cause the strap to be a leverage to push the brace upward on the leg as the patella or kneecap travels over the front of the knee. The condyle pads are fixed by a riveted type piece of plastic attachment to the inner aspects of the hinges. The lowermost, or bottom, strap is attached to a wedge that is univerally adjustable and can be attached to closely fitted flat surface medially on the subcutaneous border of the tibia so that it can gain purchase to the subcutaneous border. The outer edge of the wedge is rounded to rest against the strap and the inside engage the interior of the tibia to grasp the leg firmly by its anatomical configuration.

One of the advantages of this invention is that one size fits all even though lesser length of brace can be employed, the overall length of the composite knee brace is somewhat longer that the prior art has employed.

Fig. 1 is an isometric view showing one embodiment of this invention.

Fig. 2 is a partial cross-sectional view taken along the line II-II of Fig. 1.

Fig. 3 is a partial cross-sectional view taken along the line III-III of Fig. 1.

Fig. 4 is an isometric view of the knee brace of Fig. 1.

This invention will be described as employed in allowing the healing of a leg injury in preventing rotation and subluxation, as well as longitudinal dislocation of the tibia with respect to the femur of the leg; although it may be useful in other applications.

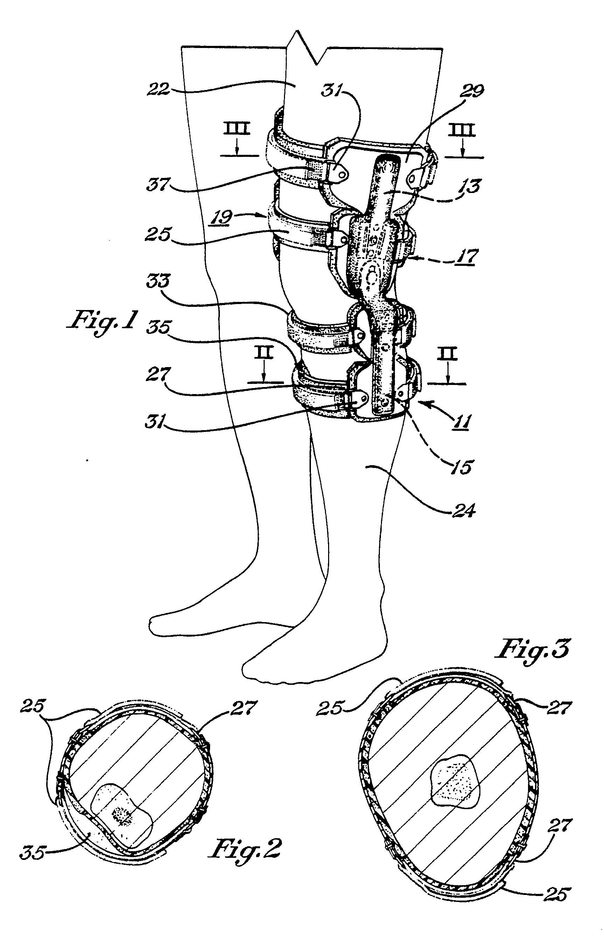

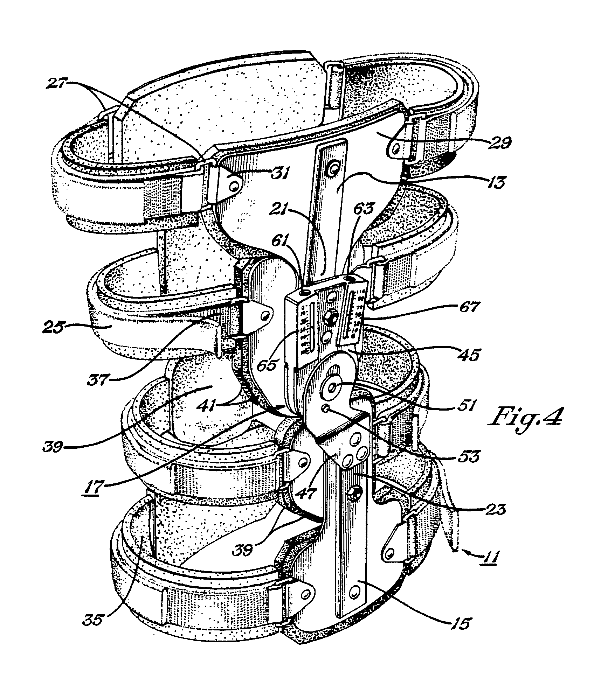

Referring initially to Fig.1, the knee brace 11 is illustrated in position for controlling the degree of motion and the lateral and longitudinal migration that is permitted by wearer's distal member with respect to its proximal member while controlling rotation and subluxation of the tibia with respect to the femur. The knee brace 11, Figs. 1 and 4, includes first and second pairs of uprights or braces 13, 15, hinge means 17 and means 19 for adjustably placing and holding the respective pairs of elongated braces 13, 15 and hinge means 17 in a desired correct attained position on opposite sides of the thigh, knee and calf of the wearer.

Each of the braces is relatively stiff so as to resist both torsion and bending forces, or loads. A preferred structural material for the core of the brace is an elongate piece of aluminum having predetermined width and thickness; for example, about 2 centimeters wide and about 3 millimeters thick. The respective braces have their central ends, 21, 23, Fig. 4, connected with their respective hinge means 17. The braces are adapted to be positioned on opposite sides of the wearer's thigh and calf. As illustrated, the first pair of braces are adapted to be placed on respective sides of the wearer's thigh 22. The second pair of braces are adapted to be emplaced along respective sides of the wearer's calf 24.

Any other structurally strong bracing material can be employed. The type braces employed in the prior art may be employed herein and the respective braces, whether or not they are affixed to plastic or other materials, such as Velcro, for being held in place, will depend on the type of means 19 for holding them in place and upon the hinge means 17.

The hinge means 17 may comprise any of the satisfactory hinge means of the prior art. Preferably, the hinge means 17 will be a complex hinge that is able to accomodate simulated motion of an actual knee requiring a compound center of rotation, such as described in the aforementioned US-A- 4 489 718, inventor Kelsey (no middle initial) Martin, filed 3/8/83 and assigned to the assignee of this invention; and the descriptive matter of that patent application is incorporated herein by reference for details that are omitted herefrom. In that application, there was disclosed means for limiting the degrees of pivotal movement in extension and flexion of the calf with respect to the thigh, and hence of the tibia with respect to the femur. Specifically, the hinge means of that application had indices and an indicator that could be screwed to limit the range of motion that the wearer would have. Of course, other types of hinges have been known in the prior art and as long as they are satisfactory to the doctor employing the knee brace of this invention, they may be employed. As described in the aforementioned patent US-A-4,407,276, "BRACE FOR ARTICULATED LIMBS", the type of hinge means is not critical to this invention. It should be compatible with the means 19 for holding the braces in place, however.

The means 19 for placing and holding the braces in a correct desired attained position comprise, in this invention, a plurality of inelastic, non-metallic straps that are padded on their interior for comfort and that can be tightened to the desired degree to prevent rotation and subluxation of the tibia with respect to the femur, as well as prevent and control distal migration and compensate for laxity of the ligaments in the leg.

In accordance with this invention, Figs. 1-4, the plurality of straps 25 are connected, as by a way that includes D-ring means 27 at both the anterior and posterior of the respective braces. As illustrated, semi-rigid plastic pieces 29 are connected with the respective braces 13 and 15, as by being riveted thereto, and are in turn riveted to the straps 31 on the D-ring means 27. In fact, as illustrated, there are plurality of 8 anterior D-ring means and 8 posterior D-ring means to which respectively, 4 anterior straps and 4 posterior straps are connected in fully adjustable manner so as to be able to be tightened to any desired degree of tension. Expressed otherwise, the D-ring means affixing the respective straps are located on the anterior of the braces above and below the hinges and on the posterior of the braces above and below the hinges. The connection of the D-ring means is indirectly through the rigid one or more pieces of plastic 29. A plurality of respective pads 33 are interposed interiorly of the respective straps 25 for comfort when the straps are tightened to the desired snuggness of fit. In the earlier application such as the aforementioned US-A-4,407,276 and US-A-4 489 718, the means 19 included a flexible sheet of cushion material that is adapted to be wrapped snugly around the wearer's thigh and the wearer's calf with a preferred type of flexible material being medium density, open cell polyurethane foam having a thickness of about 3-8 (3/8) inch. This type of flexible sheet of cushion material can be employed in this invention although with pads 33 interiorly of the straps 25, the sheets are not necessary; and more nearly direct engagement of the respective tibia wedge and the condyle pads can be achieved by using the straps 25 with their respective individual D-ring means 27 for attaching them and for tightening to the desired tightness front and back to complete the encirclement of the thigh or the calf of the wearer. Ordinarily these straps include Velcro tabs such that they can be affixed on one side and then pulled through to the desired adjustment and folded back to fasten to the Velcro tabs. The straps also hold the padded wedge 35, Figs. 1 and 2, in place adjacent the interior front of the tibia to prevent rotation. The Velcro may be seen by the reference numeral 37, Fig. 1 onto which the respective straps ends are folded back for fastening. As is well recognized, the Velcro and related material comprise a series of J-shaped hooks that engage loops and hold a desired attained position or tautness.

The respective straps and their coacting and co-engaging D-ring means, together with their tabs, plastic, and metallic braces or hinges, enable complete encirclement of the leg of the wearer at either the thigh or the calf and have the advantages delineated hereinafter. The primary improvement of one aspect of this invention is the use of the flexible, inelastic straps that are fully adjustable and are adapted to fit any size leg with the attaching at the anterior and posterior with their respective sets of D-ring means for affixing the straps above and below the hinge means; in combination with the plurality of pads disposed interiorly of and connected with the straps and the pairs of braces so as to control subluxation, rotation and distal migration of the tibia. The pads 33, as well as the condyle pads 39, 41, are preferably formed of a resilient material. An excellent material has been found to be closed cell polyurethane which forms a superior foam pad. Of course, other forms of resilient material such as foam rubber and the like could be employed if desired.

The hinge means 17 includes a single cam slot operator, a first member having a slot simulating the path of traversal in flexure of the wearer's knee and at least one second member having a cam means engaging the slot so as to traverse the slot in flexion or extension flexural motion. The first member and at least one second member co-engage each other and are adapted to move pivotally with respect to each other about a central shaft 31 engaging the respective aperture and slot, as described in the afore mentioned US-A-4 489 718 in more nearly complete detail. As illustrated, and preferably, the hinge means 17 includes two second members 47 sandwiching therebetween a first member 45, Fig. 4 with a slot extending completely through the first member. In this way the cam comprises a pin shaft that slidably engages the slot yet locks the hinge together to restrict movement of the respective member with respect to each other and limit the flexion and extension of the wearer's lower leg, or distal member with respect to the femur. The positive lock for limiting the range of flexion comprises flexible plunger means; such as springs (not shown); that are movably disposed in the respective ends of the slot for restricting movement of the cam; and threaded nuts; such as Allen-head nuts; for positioning the limits. Specifically, the slots terminate in respective threaded bores 61, 63, Fig. 4, and, respective, threaded nuts (not shown) are rotatably screwed into the respective threaded bores for moving the flexible plungers, or springs. Each of the nuts has an associate indicator that is moved longitudinally of indices 65, 67. As indicated, degrees are shown ranging, inversely from 0 to 110° of motion. Preferably the respective indicators may be of anodized aluminum; such as, red anodized aluminum or the like. If desired, they can be rotatably affixed to the respective nuts or plungers. Ordinarily, such affixing is unnecessary, since the plungers tend to follow and can be made to follow the nuts by flexure of the hinge means 17. The flexure causes the cam 51 to move the respective springs to maintain the indicators adjacent the respective nuts with which they are associated. This reduces the expense of a much more elaborate and respective interconnection therebetween.

The respective members of the hinge and nuts may be formed of any material. As illustrated, they are formed of aluminum to facilitate machining and the like. Of course, easily cleanable and relatively noncorrodible metal; such as, stainless steel; can be employed. In fact, mild steel or other materials can be employed, although special care must be taken to prevent corrosion or the like which can worsen the hygiene problem with such materials. If desired, on the other hand, plastic; such as, Delrin, Nylon or other machinable plastic can be employed.

In this embodiment, the bores 61, 63 are diverging at an angle in the range of 10 to 45°, preferably, 10 to 30° to facilitate access for tightening and loosening the respective nuts and indicators; yet, not so great an angle as to provide a wide hinge that would encumber operation. The angle is the angle between the central longitudinal axis of the bores and the central longitudinal axis of the brace 13 nearest thereto.

In operation, the knee brace is emplaced with the hinge adjacent the knee and the straps are tightened to the desired degree of tension about the calf and thigh of the wearer's leg. As indicated hereinbefore, the advantages of this invention is that the straps can be individually tightened to obtain the desired degree of tension and frictional engagement to prevent rotation, by way of holding the wedge 35 adjacent the anterior interior of the tibia, regardless of the size of the wearer's leg. The eight points of affixation, front and rear, with independently adjustable straps front and back control subluxation and compensate for laxity of the ligaments.

The hinges are emplaced with the internal two condyle pads on each side snugly engaging and supporting the condyles (bone knuckles) of the respective tibia and femur. The diverging slots on the hinge allow access to facilitate adjustment. A resilient cover can be emplaced over the hinge if the knee brace is to be employed in sports or the like.

Thereafter, the adjustably placed wedge and attached pads are adjusted as necessary to maintain the desired degree of control, regardless of change of circumstance, as by slimming or fattening of the leg or the like. Moreover, the ease with which the brace can be changed increases personal hygiene of the wearer.

From the foregoing it can be seen that all of the objects delineated hereinbefore have been effected.

Although this invention has been described with a certain degree of particularity, it is understood that the present disclosure is made only by way of example and that numerous changes in the details of construction and the combination and arrangement of parts may be resorted to without departing from the spirit and the scope of the invention, reference being had for the latter purpose to the appended claims.

## Claims

1. A knee brace for alleviating problems with tibia rotation, subluxation and migration with respect to a femur of the wearer, comprising:

   first and second pairs of elongate braces (13,15) with each of said braces being rotationally stiff so as to resist both torsion and bending forces, said first pair of braces (13) being adapted to lie on opposite sides of the wearer's thigh (22) and said second pair of braces (15) being adapted to lie on opposite sides of the wearer's calf (24),

   a pair of respective hinge means (17) connected respectively with the first and second pairs of braces (13,15) at their respective central ends for allowing and controlling flexion and extension of the wearer's leg, and

   means (19) for adjustably placing and holding respective pairs of elongate braces (13,15) at desired attained positions on respective opposite sides of the wearer s thigh (22) and calf (24) with said hinge means (17) positioned correctly adjacent the wearer s knee, said adjustable means (19) comprising a plurality of flexible non-metallic straps (25) connected to a plurality of respective attaching means (27) anteriorly and posteriorly of said braces (13,15),

   a plurality of pads (33,39,41) being disposed interiorly of and respectively connected with said straps (25) and said pairs of braces (13,15) for comfort of the wearer, characterized in that

   said plurality of straps (25) comprises eight inelastic straps (25) for adjustably placing and holding the respective pairs of elongate braces (13,15) in the desired position that are fully adjustable and adapted to fit any size leg in having attaching means, such as Velcro-attachments, comprising a series of J-shaped hooks that engage loops and hold a desired position,

   said plurality of attaching means (27) comprising 16 D-ring means (27) for affixing respective straps (25), 8 said D-ring means (27) being connected at the anterior of said braces (13,15) above and below said hinge means (17) and 8 said D-ring means (27) being connected at the posterior above and below said hinge means (17), said D-ring means (27) being indirectly connected with said pairs of braces (13,15) through said rigid pieces of plastic (29) defining on the inner aspect of each hinge a pair of respective coufoured coudyle pads (39,41) on each side defining respective individual points for affixing said plurality of the 8 straps (25) above and below said hinge means (17) and holding the braces in place such that one size fits any leg, controls subluxation and distal migration and compensates for laxity of ligaments in the leg, each pair of said two condyle pads (39,41) allowing each segment of the leg to flex independently of the other without bunching either said condyle pad (39,41) with respect to said condyle of either the tibia or the femur, said condyle pads (39,41) being shaped, respectively, to closely fit the supracondylar regions of the tibia and femur, respectively, such that they support the condyle regardless of flexion or extension of the tibia with respect to the femur.

2. The knee brace of claim 1, characterized in that an adjustably placed wedge (35) is attached to the bottom interior strap (25) by Velcro (37) or related material comprising a series of J-shaped hooks that engage loops and hold the desired position and is movable circumferentially to a desired position for comfort and to control rotation of said tibia when said bottom interior is tightened in conjunction with said bottom posterior strap (25).

3. The knee brace of claim 2, characterized in that said condyle pads (39,41) are two respective interior contoured plastic foam pads (39,41) affixed to semi-rigid plastic (29) interiorly of two longitudinally exterior pads affixed to the same type of semi-rigid plastic (29), the semi-rigid plastic (29) being connected with said braces (13,15) and said hinge means (17) on each side of the brace so as to be held in place by said fully adjustable straps (25) to fit any wearer's leg.

4. The knee brace of claim 3, characterized in that semi-rigid plastic is connected longitudinally of said braces (13,15) with a second sheet of properly shaped plastic of same material connected interiorly of said first sheet of plastic (29) and said braces (13,15) and said hinge (17), each set of anterior straps (31) having 8 D-ring means (27) at the anterior and each set of posterior straps (31) having 8 D-ring means (27) at the posterior of said plastic (29) for affixing of said flexible inelastic straps (25) with the desired degree of tension and in that there are 8 points of fixation with the 16 respective D-ring means (27) for affixing said posterior and anterior in elastic straps (25), each strap (25) having a plastic foam pad (33) interiorly thereof for the comfort of the wearer.

5. The knee brace of claim 1, characterized in that respective plastic foam covers having 1.25 cm (1/2 inch) minimum thickness are provided for the right and left sides of the respective hinge means (17) and respective braces (13,15) such that said knee brace can be worn in sports activities without injury to other players.

6. The knee brace of claim 1, characterized in that each said hinge means (17) has a single engaged cam and slot for simulating flexural motion of the wearer's knee and has a positive lock in the form of respective flexible plunger means that are movably disposed in respective ends of respective longitudinal slots and terminate in respective threaded bores (61,63) with respective threaded nuts that are rotatably screwable into said respective threaded bores (61,63) for moving said flexible plungers for restraining the movement of said cam, said bores (61,63) diverging outwardly at an angle to facilitate access to the respective threaded nuts, said threaded nuts having associated indices (65,67) and indicators for indicating the degrees of flexural motion permitted by said nuts and plungers.

7. The knee brace of claim 6, characterized in that said bores (61,63) diverge at an angle in the range of 10-45° with respect to the central longitudinal axis of the brace (13,15) connected most closely adjacent said bores (65,67).

8. The knee brace of claim 7, characterized in that said bores (61,63) diverge at an angle in the range of 10-30° with respect to the central longitudinal axis of the brace (13,15) connected most closely adjacent said bores (65,67).

**Revendications**

1. Orthèse pour genou destinée a atténuer les problèmes concernant la rotation, la subluxation et la migration du tibia par rapport au fémur du porteur de l'orthèse, comprenant :

des première et seconde paires d'armatures orthopédiques allongées (13, 15), chacune des armatures étant rigide en rotation afin de résister, à la fois, aux forces de torsion et de flexion, la première paire d'orthèses (13) étant conçue pour venir se disposer sur les côtés opposés de la cuisse (22) du porteur et la seconde paire d'orthèses (15) étant conçue pour venir se disposer sur les côtés opposés du mollet (24) du porteur ;

une paire de moyens d'articulation respectifs (17), reliés respectivement aux première et seconde paires d'orthèses (13, 15) à leurs extrémités centrales respectives, afin de permettre et de régler la flexion et l'extension de la jambe du porteur ;

un moyen (19) destiné à mettre et maintenir en ajustement les paires respectives des orthèses allongées (13, 15), à des positions désirées, localisées sur les cotés respectifs de la cuisse (22) et du mollet (24) du porteur, les moyens d'articulation (17) étant positionnés correctement en position adjacente au genou du porteur, le moyen réglable (19) comprenant plusieurs bandes (25) flexibles non métalliques, reliées à plusieurs moyens respectifs de fixation (27) à l'avant et a l'arrière des orthèses (13, 15),

plusieurs rembourrages (33, 39, 41) étant disposés à l'intérieur de et combinés respectivement aux bandes (25) et aux paires d'orthèses (13, 15) pour le confort du porteur, **caractérisée en ce que**

l'ensemble des bandes (25) est constitué par huit bandes non élastiques (25) destinées à placer en ajustement et à maintenir les paires respectives d'armatures orthopédiques allongées (13, 15), dans la position désirée, qui sont complètement réglables et conçues pour s'adapter à n'importe quelle dimension de jambe, par le fait qu'elles sont munies de moyens de fixation, tels que des fixations Velcro, comprenant une série de crochets en forme de J qui viennent s'engrener dans des boucles et qui maintiennent une position désirée,

l'ensemble des moyens de fixation (27) comprenant seize moyens d'anneaux en D (27) destinés à fixer les bandes respectives (25), huit moyens d'anneaux en D (27) étant reliés à l'avant des armatures métalliques (13, 15) au-dessus et en dessous des moyens d'articulation (17) et huit de ces moyens d'anneaux en D (27) étant reliés à l'arrière au-dessus et en dessous des moyens d'articulation (17), les moyens d'anneaux en D (27) étant reliés indirectement aux paires d'armatures orthopédiques (13, 15) par l'intermédiaire d'éléments rigides en plastique (29) qui définissent, sur la face interne de chaque articulation et de chaque côté, une paire de rembourrages respectifs configurés (39, 41) pour condyles, et qui définissent des points individuels respectifs, destinés à fixer l'ensemble des huit bandes (25) au-dessus et en dessous des moyens d'articulation (17) et à maintenir les orthèses en place, de telle sorte qu'une seule dimension s'adapte à n'importe quelle jambe, jugule la subluxation et la migration distale et compense l'hyperlaxité ligamentaire dans la jambe, chaque paire des deux rembourrages pour condyles (39, 41) permettant à chaque segment de la jambe de fléchir indépendamment l'un de l'autre, sans provoquer le tassement du rembourrage pour condyles (39, 41) soit par rapport aux condyles, soit par rapport au tibia ou au fémur, les rembourrages pour condyles (39, 41) étant façonnés respectivement pour venir se disposer en ajustage serré contre les zones suscondyliennes du tibia et du fémur, respectivement, de telle sorte qu'ils supportent le condyle quelle que soit la flexion ou l'extension du tibia par rapport au fémur.

2. Orthèse pour genou selon la revendication 1, **caractérisée en ce qu'**un coin (35) placé de manière réglable est fixé à la bande antérieure inférieure (25) par du Velcro (37) ou une matière apparentée, comprenant une série de crochets en forme de J qui viennent s'engrener dans des boucles et qui maintiennent la position désirée, le coin pouvant se déplacer circonférentiellement pour atteindre une position désirée et procurer du confort, ainsi que pour maîtriser la rotation du tibia lorsqu'on serre la bande inférieure antérieure, conjointement, avec la bande inférieure postérieure (25).

3. Orthèse pour genou selon la revendication 2, **caractérisée en ce que** les rembourrages pour condyles (39, 41) sont constitués par deux rembourrages respectifs internes (39, 41), façonnés en plastique mousse, fixés à un élément semi-rigide en plastique (29) à l'intérieur de deux rembourrages externes s'étendant longitudinalement, fixés au même type d'élément semi-rigide en matière plastique (29), l'élément semi-rigide en matière plastique (29) étant combiné aux armatures orthopédiques (13, 15) et au moyen d'articulation (17) de chaque côté de l'orthèse, de façon à être maintenu en place par les bandes (25) complètement réglables, afin de s'adapter à n'importe quelle jambe du porteur d'une orthèse.

4. Orthèse pour genou selon la revendication 3, **caractérisée en ce que** le plastique semi-rigide est joint longitudinalement aux armatures orthopédiques (13, 15), une seconde feuille mince de plastique du même matériau, façonnée de manière appropriée, étant disposée entre la première feuille de plastique (29), et les armatures orthopédiques (13, 15) et l'articulation (17), chaque série de bandes antérieures (31) étant munie de huit moyens d'anneaux en D (27) à l'avant du plastique (29) et chaque série de bandes postérieures (31) étant munie de huit moyens d'anneaux en D (27) à l'arrière du plastique (29), afin de fixer les bandes flexibles non élastiques (25) avec le degré désiré de tension et **en ce que** l'on prévoit huit points de fixation avec les seize moyens respectifs d'anneaux en D (27) afin de fixer les bandes non élastiques antérieure et postérieure (25), chaque bande (25) étant munie, sur sa face intérieure, d'un rembourrage en mousse plastique (33) pour le confort du porteur de l'orthèse.

5. Orthèse pour genou selon la revendication 1, **caractérisée en ce qu'**on prévoit des recouvrements respectifs en mousse plastique ayant 1,25 cm (1/2 pouce) d'épaisseur minimale pour les côtés droit et gauche des moyens d'articulation respectifs (17) et pour les armatures orthopédiques respectives (13, 15), de telle sorte que l'orthèse pour genou puisse être portée dans des activités sportives sans provoquer de blessures à d'autres joueurs.

6. Orthèse pour genou selon la revendication 1, **caractérisée en ce que** chaque moyen d'articulation (17) est muni d'une fente et une came unique dans laquelle elle est engrenée, afin de simuler le mouvement de flexion du genou du porteur de l'orthèse et est muni d'un blocage positif sous forme de moyens de poussoirs flexibles respectifs, qui sont disposés de manière mobile, dans les extrémités respectives de fentes longitudinales respectives et qui se terminent dans des alésages filetés respectifs (61, 63), des écrous filetés respectifs pouvant être vissés en rotation au sein des alésages filetés respectifs (61, 63) afin de déplacer les poussoirs flexibles dans le but de restreindre le mouvement de la came, les alésages (61, 63) s'écartant vers l'extérieur en formant un angle afin de faciliter l'accès aux écrous filetés respectifs, les écrous filetés étant munis d'indices associés (65, 67) et d'indicateurs destinés à indiquer les degrés de mouvement de flexion autorisés par les écrous et les poussoirs.

7. Orthèse pour genou selon la revendication 6, **caractérisée en ce que** les alésages (61, 63) s'écartent en formant un angle dans le domaine de 10 à 45° par rapport à l'axe longitudinal central de l'armature orthopédique (13, 15) disposée le plus près des alésages (61, 63) en position adjacente à ces derniers.

8. Orthèse pour genou selon la revendication 7, **caractérisé en ce que** les alésages (61, 63) s'écartent en formant un angle dans le domaine de 10 à 30° par rapport à l'axe longitudinal central de l'armature orthopédique (13, 15) disposée le plus près des alésages (61, 63) en position adjacente à ces derniers.

**Patentansprüche**

1. Kniestütze zum Mildern von Problemen aufgrund von Schienbeinrotation, Subluxation und Migration in bezug auf einen Oberschenkel des Trägers, mit:

einem ersten und einem zweiten Paar langgestreckter Bügel (13, 15), wobei jeder Bügel drehsteif ist, so daß er sich sowohl Torsions- als auch Biegekräften widersetzt, wobei das erste Paar Bügel (13) auf entgegengesetzten Seiten des Oberschenkels (22) des Trägers und das zweite Paar Bügel (15) auf entgegengesetzten Seiten der Wade (24) des Trägers zu liegen kommt,

einem Paar Scharniereinrichtungen (17), die mit dem ersten bzw. zweiten Paar Bügeln (13, 15) an deren zentralen Enden verbunden sind, um die Beugung und Streckung des Beins des Trägers zu gestatten und zu kontrollieren, und

einer Einrichtung (19) zum einstellbaren Plazieren und Halten der Paare langgestreckter Bügel (13, 15) in gewünschten erreichten Positionen auf den entgegengesetzten Seiten des Oberschenkels (22) und der Wade (24) des Trägers bei korrekt an dem Knie des Trägers positionierten Scharniereinrichtungen (17), wobei die einstellbare Einrichtung (19) mehrere flexible, nichtmetallische Bänder (25) aufweist, die mit mehreren Befestigungseinrichtungen (27) vorn und hinten an den Bügeln (13, 15) verbunden sind,

mehreren Polstern (33, 39, 41), die innen an den Bändern (25) angeordnet und mit diesen und den Bügelpaaren (13, 15) für den Komfort des Trägers verbunden sind, dadurch gekennzeichnet, daß

die Bänder (25) acht unelastische Bänder (25) zum einstellbaren Plazieren und Halten der Paare langgestreckter Bügel (13, 15) in der gewünschten Position umfassen, die vollständig einstellbar sind und für jede Beingröße passen und Befestigungsmittel wie zum Beispiel Klettverschlußbefestigungen aufweisen, die aus einer Reihe von J-förmigen Haken bestehen, welche in Ösen einfassen und eine gewünschte Position beibehalten,

wobei die Befestigungseinrichtungen (27) 16 D-Ringeinrichtungen (27) zum Befestigen der Bänder (25) umfassen, von denen 8 D-Ringeinrichtungen (27) an der Vorderseite der Bügel (13, 15) oberhalb und unterhalb der Scharniereinrichtungen (17) angeschlossen sind und von denen 8 D-Ringeinrichtungen (27) an der Rückseite oberhalb und unterhalb der Scharniereinrichtungen (17) angeschlossen sind, wobei die D-Ringeinrichtungen (27) mit den Bügelpaaren (13, 15) über starre Kunststoffteile (29) indirekt verbunden sind, welche auf der Innenseite jedes Scharniers ein Paar profilierter Gelenkhöckerpolster (39, 41) auf jeder Seite bilden, einzelne Punkte zur Befestigung der Anzahl der 8 Bänder (25) oberhalb und unterhalb der Scharniereinrichtungen (17) bilden und die Bügel in ihrer Lage halten, so daß eine Größe an jedes Bein paßt, Subluxation und distale Migration steuert und Lockerheit von Bändern in dem Bein kompensiert, wobei jedes Paar der beiden Gelenkhöckerpolster (39, 41) gestattet, jeden Abschnitt des Beins unabhängig von dem anderen zu beugen, ohne jedes Gelenkhöckerpolster (39, 41) in bezug auf den Gelenkhöcker entweder des Schienbeins oder des Oberschenkels zusammenzuballen, wobei die Gelenkhöckerpolster (39, 41) so geformt sind, daß sie Bereichen des Schienbeins und des Oberschenkels oberhalb der Gelenkhöcker eng angepaßt sind, so daß sie den Gelenkhöcker ungeachtet der Beugung oder Streckung des Schienbeins in bezug auf den Oberschenkel abstützen.

2. Kniestütze nach Anspruch 1, dadurch gekennzeichnet, daß ein einstellbar angeordneter Keil (35) an dem unteren inneren Band (25) mittels Klettverschluß (37) od. dgl. Material, das eine Reihe von J-förmigen Haken aufweist, die in Ösen einfassen und die gewünschte Position beibehalten, befestigt und in Umfangsrichtung in eine gewünschte Position für den Komfort und zum Kontrollieren der Drehung des Schienbeins bewegbar ist, wenn die untere Innenseite in Verbindung mit dem unteren hinteren Band (25) angezogen wird.

3. Kniestütze nach Anspruch 2, dadurch gekennzeichnet, daß die Gelenkhöckerpolster (39, 41) zwei innere, profilierte Schaumstoffpolster (39, 41) sind, die an dem halbsteifen Kunststoff (29) innerhalb von zwei longitudinal äußeren Polstern befestigt sind, welche an demselben Typ von halbsteifem Kunststoff (29) befestigt sind, wobei der halbsteife Kunststoff (29) mit den Bügeln (13, 15) und den Scharniereinrichtungen (17) auf jeder Seite des Bügels verbunden ist, so daß er durch die vollständig einstellbaren Bänder (25) in seiner Lage gehalten wird, um sich jedem Bein des Trägers anzupassen.

4. Kniestütze nach Anspruch 3, dadurch gekennzeichnet, daß der halbsteife Kunststoff in Längsrichtung der Bügel (13, 15) mit einer zweiten Platte passend geformten Kunststoffes aus dem gleichen Material verbunden ist, das innerhalb der ersten Platte aus Kunststoff (29) und der Bügel (13, 15) und des Scharniers (17) angeschlossen ist, wobei jeder Satz vorderer Bänder (31) 8 D-Ringeinrichtungen (27) auf der Vorderseite und jeder Satz hinterer Bänder (31) 8 D-Ringeinrichtungen (27) an der Rückseite des Kunststoffes (29) hat zum Befestigen der flexiblen, unelastischen Bänder (25) mit dem gewünschten Grad an Zugspannung, und daß es 8 Befestigungspunkte mit den 16 D-Ringeinrichtungen (27) zum Befestigen der vorderen und hinteren unelastischen Bänder (25) gibt, wobei jedes Band (25) ein Schaumstoffpolster (33) an seiner Innenseite für den Komfort des Trägers hat.

5. Kniestütze nach Anspruch 1, dadurch gekennzeichnet, daß Schaumstoffabdeckungen, die eine Mindestdicke von 1,25 cm (1/2 Zoll) haben, für die rechte und linke Seite der Scharniereinrichtungen (17) und der Bügel (13, 15) vorgesehen sind, so daß die Kniestütze bei sportlichen Aktivitäten getragen werden kann, ohne daß andere Spieler verletzt werden.

6. Kniestütze nach Anspruch 1, dadurch gekennzeichnet, daß die Scharniereinrichtungen (17) jeweils einen einzelnen, im Eingriff befindlichen Nocken und Schlitz zum Simulieren der Beugebewegung des Knies des Trägers und eine formschlüssige Verriegelung in Form von biegsamen Stempeleinrichtungen haben, welche in den Enden der Längsschlitze beweglich angeordnet sind und in Gewindebohrungen (61, 63) endigen, wobei mit Gewinde versehene Muttern drehbar in die Gewindebohrungen (61, 63) schraubbar sind, um die flexiblen Stempel zu bewegen und die Bewegung des Nockens zu verhindern, wobei die Bohrungen (61, 63) unter einem Winkel nach außen divergieren, um den Zugang zu den mit Gewinde versehenen Muttern zu erleichtern, wobei die mit Gewinde versehenen Muttern zugeordnete Markierungen (65, 67) und Anzeiger zum Anzeigen des Grads der Beugebewegung, den die Muttern und Stempel zulassen, haben.

7. Kniestütze nach Anspruch 6, dadurch gekennzeichnet, daß die Bohrungen (61, 63) unter einem Winkel in dem Bereich von 10-45° in bezug auf die zentrale Längsachse des Bügels (13, 15) divergieren, dessen Anschluß den Bohrungen (65, 67) am nächsten ist.

8. Kniestütze nach Anspruch 7, dadurch gekennzeichnet, daß die Bohrungen (61, 63) unter einem Winkel in dem Bereich von 10-30° in bezug auf die zentrale Längsachse des Bügels (13, 15) divergieren, dessen Anschluß den Bohrungen (65, 67) am nächsten ist.

*Fig.1*

*Fig.2*

*Fig.3*

*Fig.4*